# EUROPEAN PATENT APPLICATION

(11) **EP 3 351 190 A1**
(43) Date of publication of application: **25.07.2018**
(21) Application number: 18275004.2
(22) Date of filing: 12.01.2018
(51) Int. Cl.: A61B 17/12, A61B 17/00

(54) **ANEURYSM TREATMENT APPARATUS**

(30) Priority: 20.01.2017 GB 201700991
(71) Applicant: Cook Medical Technologies LLC, Bloomington, IN 47404 (US)
(72) Inventor: Moeller, Rasmus Buch, 4100 Ringsted (DK)
(74) Representative: Williams Powell

(57) **Abstract**

Aneurysm treatment apparatus includes a steerable catheter assembly (10) having a catheter (12) with a steerable distal end (14). A pouch (40) is disposed over the distal end (14) of the catheter (12) so as to be in co-axial overlying relationship therewith. A constraining sheath (60) is disposed over a major portion of the pouch (40) and has a proximal sheath portion (64) which is bonded to the catheter (12). The sheath (60) terminates short of the distal end (26) of the catheter (12), so as to leave a distal end of the pouch (40) exposed. The steerable catheter can be steered into an aneurysm sac (80) and filler material fed through the catheter (12) in order to fill chamber (50) of the pouch (40). The pouch (40) will expand from its distal end (44) with filler material and gradually slide off the sheath (12) as it fills the aneurysm sac (80). The sheath (60) prevents the pouch (40) from expanding proximally, and potentially adversely affecting the filling process.

## Description

### Technical Field

The present invention relates to apparatus for filling a cavity in a patient, particularly a saccular aneurysm.

### Background Art

Many factors contribute to the formation of saccular aneurysms, particularly vascular aneurysms. One of the major contributors is wall shear stress (WSS), which in addition to hypertension leads to a reduction in the elastic tissue of the tunica media, thereby contributing to the formation of the aneurysm. The effect of wall shear stress is heightened at certain anatomical geometries such as bifurcations, for instance at the point where the basilar artery (BA) divides into the posterior cerebral arteries (PCA). Aneurysms also occur in the wall of vessels.

If left untreated, persistent pressure from the blood flow on the weakened wall tissue can lead to rupture of the vessel and consequential haemorrhaging. Treatments for aneurysms have tended to focus on reducing the pressure on the weakened vessel, for instance by diverting blood flow or by isolating the weakened part of the vessel, for example by means of a stent graft. Another treatment method involves filling the aneurysm sac with a filler material, which stops the flow of blood into the sac and as a result stops or substantially reduces the pressure on the weakened walls. An embolization coil may be used for this purpose, which causes blood statis within the interstices of the coil and as a consequence clotting of the blood, which then forms a protective barrier to blood flow into the aneurysm reducing pressure and helping to prevent vessel rupture. In other methods, the aneurysm may be filled with a biocompatible material, such as a hydrogel or a polysaccharide fibre, which may be of a biodegradable nature. A biodegradable filler performs an equivalent function to that of an embolization coil, that is to fill the aneurysm sac and provide pressure protection to the weakened vessel walls, with the additional advantage of allowing remodelling of the vessel wall over time. Moreover, biodegradation of the filler will ensure that no foreign matter remains in the patient's vessel after conclusion of the treatment.

Often, it is preferable to treat an aneurysm by filling the sac. However, complications can arise, such as migration of the filler material into the main vessel due to the force of blood flow. While this can be mitigated in part by means of a blocking device such as a stent placed at the aneurysm neck, this adds a further step to the medical procedure and leaves a foreign object in the patient.

Difficulties can also arise in terms of ensuring accurate deployment of the filler into the aneurysm sac, particularly when the neck of the sac is small or at an angle to the vessel.

Devices are known which include a pouch intended to hold the filler material. While these can increase the range of filler materials which may be used and can reduce the risk of loss of filler material into the patient's bloodstream, they do not necessarily resolve all of the disadvantages mentioned above.

Examples of known aneurysm filler apparatus having a fillable pouch are described in US-2003/0220666, US-2008/0228259, US-2006/0079923, US-2014/0188153, US-6,350,270 and US-7,666,220.

### Summary of the Invention

The present invention seeks to provide improved aneurysm treatment apparatus and method of treating aneurysms, particularly but not solely for saccular aneurysms.

According to an aspect of the present invention, there is provided apparatus for filling a cavity in a patient, the apparatus including:
an elongate catheter having a catheter distal end and a tip at an extremity of the catheter distal end, at least a part of the catheter distal end being steerable;
a steering mechanism connected to the steerable part of the catheter distal end; and
a pouch having an open proximal pouch end, a closed distal pouch end, a pouch length between the proximal and distal pouch ends, and a pouch wall forming a pouch chamber, the pouch along its length being slidably disposed in coaxial overlying relationship on the catheter distal end with the distal pouch end being adjacent and overlying the catheter tip, the pouch being steerable with the catheter distal end, the pouch being separable from the catheter;
the catheter having a lumen extending to the catheter distal end and in communication with the pouch chamber.

The apparatus provides a structure which can be positioned more reliably into a saccular aneurysm, for example, and which is also able to navigate better through tortuous vasculature and into narrow aneurysm necks. The arrangement of the pouch over the catheter ensures that the pouch can be steered with the catheter through this process.

In the preferred embodiment, the apparatus includes a sheath or sleeve attached to the catheter and extending over at least a part of the pouch on the catheter. The sheath acts to hold the pouch in place over the catheter and assists in ensuring correct expansion of the pouch into a saccular aneurysm with minimised risk of the pouch being expanded outside the aneurysm, as is possible with some prior art systems.

Advantageously, the sheath includes a sheath proximal end and a sheath distal end, wherein the sheath proximal end is attached to the catheter and the sheath distal end extends over the pouch. The sheath proximal end preferably has a smaller diameter than a diameter of the sheath distal end, and has a conical portion extending between the two ends of the sheath.

It is preferred that the sheath distal end is longitudinally spaced from the catheter tip. This allows the distal end of the pouch to expand while still on the catheter, while keeping more proximal portions of the pouch radially constrained. This can help ensure that the pouch expands only into the aneurysm sac.

In a practical example, the sheath distal end terminates at least 1cm from the catheter tip. In some embodiments, and applicable to all aspects of the present invention, this may be less than 1 cm.

The sheath may be fixed to the catheter by heat bonding, although any other type of fixation may be used including, for example, adhesive bonding, welding and so on.

Advantageously, the sheath is formed of a flexible material. This allows the sheath to curve with the distal end of the catheter during steering.

The proximal end of the pouch is advantageously coupled to the catheter substantially in fluid tight manner. This enables the pouch to be filled with a liquid, for instance.

In practice, filler material fed through the catheter lumen into the pouch will create pressure in the pouch, which will urge it to slide off the catheter. This is a more convenient way of releasing the pouch compared to prior art devices and is made possible by the arrangement of the components as taught herein.

Preferably, the proximal end of the pouch is sealable in a substantially closed state by a valve including one or valve leaflets or by an elastic membrane. The pouch may be completely sealable, although in some embodiments the proximal end of the pouch may retain a small opening not big enough to allow significant loss of filler material.

The pouch is preferably formed of one or more of:
biodegradable material;
a material which promotes cell growth;
an inelastic material.

The filler material may similarly be a biodegradable material and/or a material which promotes cell growth. In some embodiments, the filler material may be curable at body temperature. In an embodiment, the filler material is in the form of a solution or other liquid deliverable to the pouch chamber through the lumen of the steerable catheter. The filler material may comprise cyanoacrylate, GelFoam, GluBran, polyethylene glycol diacrylate, a highly viscous liquid such as silicon, fat and the like, a multi composite glue, coagulated blood and trigger such as alcohol or a thrombin.

In some embodiments, the filler material may include a first material located within the pouch chamber. The first material may be in dry form. In some embodiments, the first material includes an alginate or a super absorbent polymer. Suitable superabsorbent polymers include polyacrylamide, polyacrylate, polysaccharides, proteins and derivatives thereof.

Advantageously, the filler material includes a fluid deliverable to the pouch chamber through the lumen of the steerable catheter.

According to another aspect of the present invention, there is provided apparatus for filling a cavity in a patient, the apparatus including:
an elongate catheter having a catheter distal end and a tip at an extremity of the catheter distal end;
a pouch having an open proximal pouch end, a closed distal pouch end and a pouch chamber, the pouch being disposed in coaxial overlying relationship on the catheter distal end with the distal pouch end adjacent the catheter tip;
the catheter having a lumen extending to the catheter distal end and in communication with the pouch chamber; and
a sheath attached to the catheter and extending over at least a part of the pouch on the catheter.

The sheath preferably includes a sheath proximal end and a sheath distal end, wherein the sheath proximal end is attached to the catheter and the sheath distal end extends over the pouch.

The sheath distal end is advantageously longitudinally spaced from the catheter tip, for example terminating at least 1 cm from the catheter tip.

The skilled person will appreciate that this aspect of the present invention provides apparatus having advantages over the art without necessarily using a steerable catheter.

According to another aspect of the present invention, there is provided a method of filling cavity of a patient, including the steps of:
deploying endoluminally in a patient apparatus for filling a medical cavity, the apparatus including an elongate steerable catheter having a catheter distal end and a tip at an extremity of the catheter distal; a steering mechanism operable to steer the catheter distal end; and a pouch having an open proximal pouch end, a closed distal pouch end and a pouch chamber, the pouch being disposed in coaxial overlying relationship on the catheter distal end with the distal pouch end adjacent the catheter tip; the catheter having a lumen extending to the catheter distal end and in fluid communication with the pouch chamber;
steering the catheter distal end to a medical cavity in the patient and the pouch into the medical cavity;
supplying filler material into the pouch through the lumen to cause the pouch to expand in the cavity; and
separating the pouch from the catheter so as to leave the pouch in the cavity.

Preferably, the method includes the step of sliding the pouch distally along the catheter as it is filled.

The method advantageously includes the steps of:
navigating the steerable catheter to an aneurysm site;
operating a steering mechanism of the catheter to bend the distal end of the catheter into the aneurysm before filling the pouch with filler material.

Other features and advantageous of the invention taught herein will be apparent from the following description and accompanying drawings.

### Brief Description of the Drawings

Embodiments of the present invention are described below, by way of example only, with reference to the accompanying drawings, in which:
Figure 1 is a schematic diagram of an example of steerable catheter suitable for the present invention;
Figures 2 to 5 show an embodiment of steps of assembling the components of the preferred embodiment of apparatus, with Figure 5 showing the distal end of the apparatus in fully assembled form;
Figure 6 shows an embodiment of valve mechanism for the pouch taught herein, in various stages of deployment;
Figures 7 and 8 are schematic views showing another embodiment of suitable valve closure for the proximal end of the pouch;
Figures 9 and 10 are schematic views showing yet another embodiment of suitable valve closure for the proximal end of the pouch; and
Figures 11 to 15 show the apparatus in use in filling a saccular aneurysm.

### Description of the Preferred Embodiments

The drawings show in schematic form the principal elements of the apparatus disclosed herein, the process of assembly of the apparatus and of its usage in occluding a saccular aneurysm. Only the principal components of the apparatus are shown in the drawings and those which are not shown are commonplace in the art so will be immediately apparent to the skilled person.

The drawings are not to scale and the elements are generally shown in enlarged form for clarity of representation. The skilled person will be familiar with apparatus of this nature and the typical dimensions which it could be expected to have, which will be dependent upon the particular medical application, such as vessel or organ to be treated.

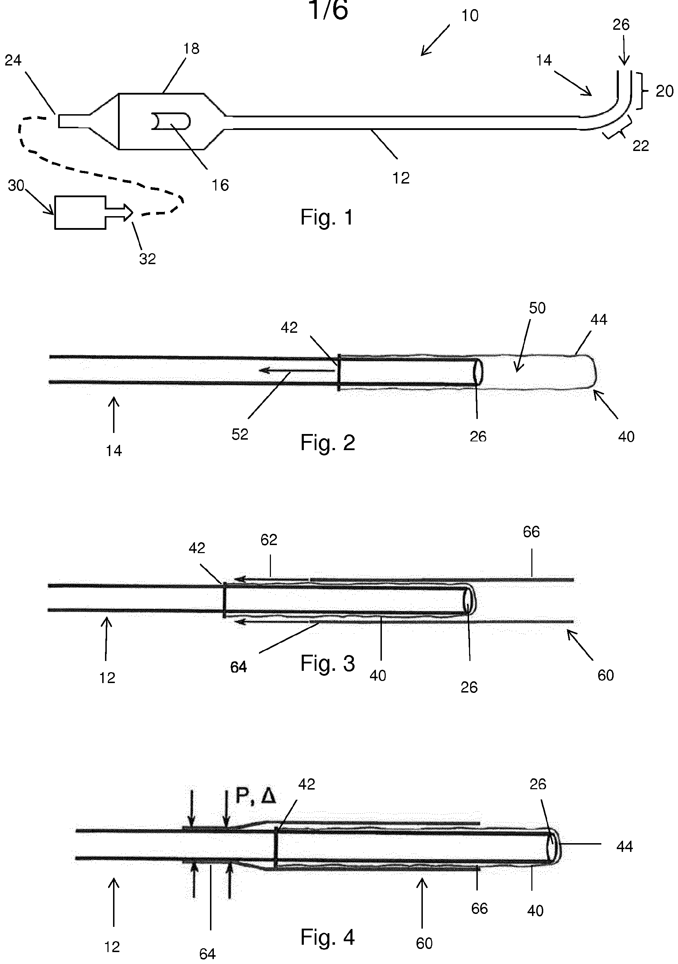

With reference first to Figure 1, this shows in schematic form a steerable catheter assembly 10 which could implement the present invention taught herein. The assembly 10 includes an elongate catheter 12, which may have a length of a few tens of centimetres to significantly more in most applications. The catheter 12 is generally flexible and includes at least one lumen passing through the length of the catheter. At its distal end 14, the catheter 12 has a steerable section which enables the distal end 14 to be selectively curved, or straightened, so as to guide the distal end 14 and thereby the catheter 12 through a patient's vasculature.

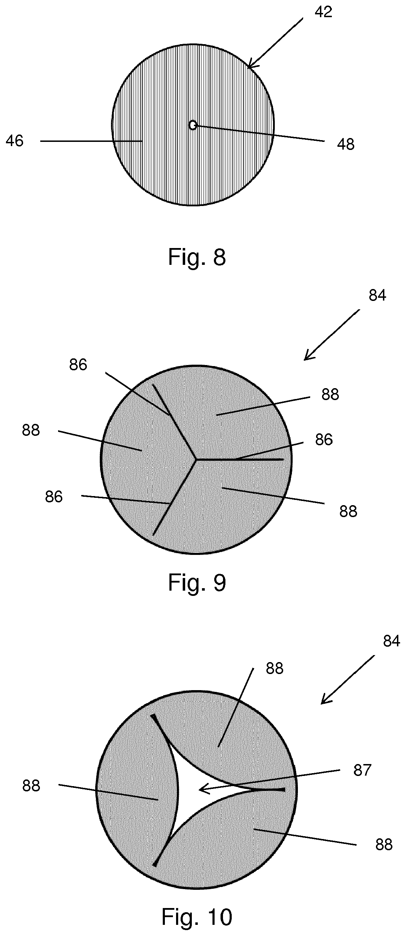

A variety of steering mechanisms for this purpose are known in the art and examples can be found in the Applicant's earlier patent applications US-2016/0302650 and US-2016/0271366, the disclosures of which are incorporated herein by reference. The teachings herein are not constrained to any particular steering mechanism and therefore any of the known mechanisms or otherwise suitable mechanisms could be used for the assembly of Figure 10. In practice, the steering mechanism will include an actuator 16 which may conveniently be coupled to a proximal handle assembly 18 of generally conventional form. The actuator 16 will be chosen upon basis of the steering mechanism used and again this will be of a structure which will be readily apparent to the skilled person, including those described in the above-mentioned patent publications of the Applicant.

In some embodiments, the entirety of the distal section 14 of the catheter 12 may be steerable, that is curvable by the steering mechanism. In other embodiments, though, the catheter 12 may include a distal tip portion 20 that is not steerable, that is, which will generally retain a straight configuration and such that only a more proximal tip portion 22 can be steered. This enables the assembly 10 to be used with a relatively stiff distal tip structure, of which an example is described below. It is preferred, though, that the entirety of the distal end portion 14 is of a steerable nature, including the distal tip portion 20.

The catheter assembly 10 also includes an inlet port 24 at its proximal end, which may be in the form of a Luer lock, a screw thread, or any other suitable fitting. The inlet port 24 connects to the lumen in the catheter 12 and to an outlet 26 at the distal tip of the catheter 12. The inlet port 24 is designed to be coupled to a source 30 of filler material, of which examples are given below. The source 30 will typically have a complimentary fitting 32 to the port 24 or to a suitable conduit connecting them to one another. The source 30 can be hand operated, such as with a syringe, or motor or hydraulically driven, for example. The type of source will be dependent upon the nature of the filler material to be fed through the catheter 12 and into the pouch.

Referring now to Figures 2 to 5, these show in schematic form the steps of assembling the components taught herein over the distal end 14 of the catheter 12. In Figures 2 to 5, the distal end 14 is shown in a straight configuration, which is typically optimal, though not essential, for the assembly procedure. The apparatus taught herein includes a pouch 40 which has an open proximal pouch end 42 and a closed pouch end 44. The proximal pouch end 42 is preferably provided with a closure mechanism, of which some examples are given below. It is to be understood, therefore, that the term "open" as used in connection with the pouch end 42 is intended to denote that the end 42 is able to be opened, primarily to allow the pouch 40 to be slid over the catheter 12 and to allow for the insertion of filler material into the pouch. The pouch 40 provides an internal pouch chamber 50, delimited by the walls of the pouch.

The pouch 40 is preferably made of an impermeable or substantially impermeable material. It may be made of an expandable material, such as an elastomeric material, although is most preferably made of a generally inextensible material. Example materials include: polyurethane, polyamide, polycarbonate, silicone, latex, polyethylene terephthalate (PET), Polyethylene, ultrahigh molecular weight polyethylene such as Dyneema, a woven material and so on. There are numerous other suitable materials known to the skilled person. In some preferred embodiments, the pouch 40 is made of a biodegradable material or a material that promotes cell growth. In this manner, the pouch 40 will become absorbed into the body and replaced by bodily tissue. Examples includepoly-L,D-lactide, poly-L-lactide, poly-D-lactide, poly(alpha hydroxy acid), polyglycolic acid, polylactic acid, polycaprolactone, polydioxanone, polyglucanate, polylactic acid-polyethylene oxide copolymers, tyrosine-derived polycarbonate, polyglycolide, modified cellulose, collagen, poly(hydroxybutyrate), polyanhydride, polyphosphoester, poly(amino acids), chitosan or combinations and/or derivatives thereof.

The pouch 40 may also be made of a thermoset elastomeric material, such as butyl rubber or a silicone rubber.

The pouch 40 is preferably of a shape consistent with the shape of the aneurysm sac to be treated, so often generally rounded. It may be provided in a variety of sizes (volumes), enabling a physician to select a pouch size compatible with the size of the saccular aneurysm to be treated. In this way, the filled pouch will not impart undue stretching force to the weakened vessel walls of the aneurysm.

Figures 2 to 5 show the pouch 40 arranged into an elongate tubular form. It is to be appreciated that the pouch 40 may be wrapped around the catheter 12, in a manner similar to the balloon of a balloon catheter.

Figures 2 to 5 show an optimal method of assembling the distal end components of the apparatus taught herein. It is to be understood, though, that this is a preferred embodiment and that the components may be assembled in a different manner, including in a different order.

Referring first to Figure 2, the pouch 40 is slid on to the catheter 12, from the distal catheter tip 26, in the direction of the arrow 52, until the closed distal pouch end 44 is adjacent the outlet port 26 at the catheter distal tip 26. This is shown in Figures 3 to 5. Thus, the entire length of the pouch 40 is preferably disposed in coaxial overlying relationship on the catheter 14. The advantage of this arrangement is that the entirety (or the majority) of the pouch 40 will be made to curve with the distal portion 14 when the steering mechanism 16 is actuated. This is in contrast to many prior art arrangements of fillable aneurysm pouches.

Referring now to Figure 3, in a second stage of the assembly process, a tubular sheath or sleeve 60 is slid in the direction of the arrows 62 onto the catheter 12 and over the earlier fitted pouch 40. The sheath 60 includes a proximal sheath end 64 and a distal sheath end 66. Figure 4 shows that the proximal sheath end 64 has been positioned beyond the proximal end 42 of the pouch 40 and bonded to the catheter, by application of pressure and heat. For this purpose, the sheath 60 can be made of any suitable heat formable material, such as a thermoplastics material. The sheath 60 may be made of the same material as the catheter 12 and in practice of any known catheter material. Polyurethane, polyamide and polyethylene are examples, as well as known bondable heat shrinkable materials. The sheath 60 is preferably flexible, such that in practice it will curve with curving of the steerable distal portion 14 of the catheter 12.

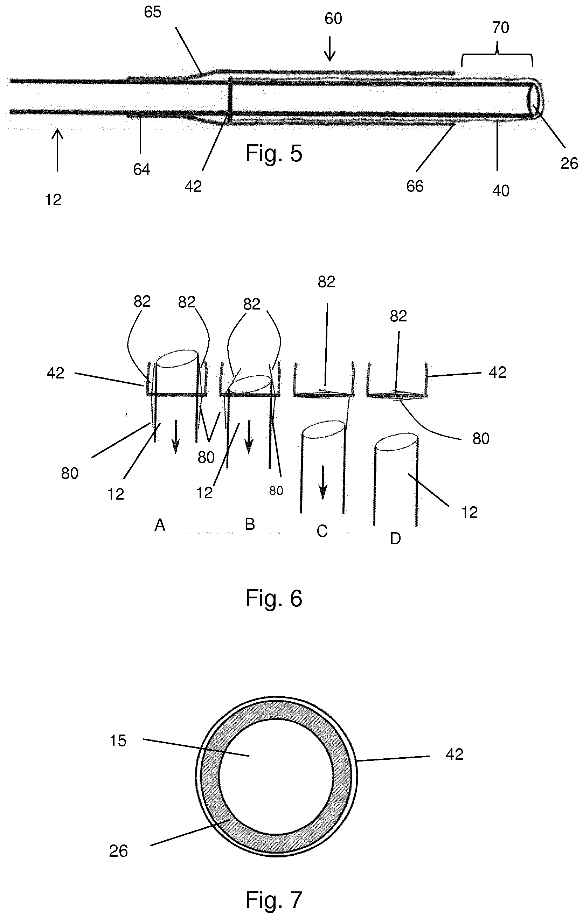

The final assembly is shown in Figure 5 and it can be seen that, in the preferred embodiment, the sheath 60 terminates short of the distal tip 26 of the catheter 12, leaving a gap 70 between its distal end 66 and the catheter tip 26. This gap 70 may be from a few millimetres up to around one centimetre or so in length, for purposes which are described detail below. Furthermore, the sheath 60 has a conical tapering section 65 between its proximal end 64 and the main, cantilevered, section of the sleeve. This section 65, which preferably has a shallow gradient, can reduce the risk of snagging and trauma to the vessel wall when the assembly 12 is removed from the patient at the end of the procedure.

The majority of the length of the pouch 40 is located within the lumen of the sheath 60 and remains constrained by the sheath. The pouch 40 is slidable on the catheter 12 and within the sheath 60.

The open proximal end 42 of the pouch 40 is preferably provided with a closure mechanism, of which two examples are shown in Figures 6 to 8. Referring first to Figure 6, this shows a mechanism in the form of two sets of valve flaps or leaflets 80, 82 disposed at the pouch open end 42. There is provided an external flap pair 80 which can swing "outwardly" and an internal flap pair 82 which can swing into the chamber 50 of the pouch 40. The flaps 80 may be made of a substantially rigid or flexible material attached to the proximal end 42 of the pouch 40 and may have a form similar to that of haemostatic or prosthetic valves which are commonplace in the art of medical devices. The flaps 80, 82 may be made of a different material to that of the pouch 40 but may equally be made of the same material as that of the pouch. Preferably, they are made of a different material as this can help minimize friction. Examples of materials could be polyethylene, polypropylene, polyamide, polyurethane, polyether block amide. Examples of biodegradable materials could be poly-L,D-lactide, poly-L-lactide, poly-D-lactide, poly(alpha hydroxy acid), polyglycolic acid, polylactic acid, polycaprolactone, polydioxanone, polyglucanate, polylactic acid-polyethylene oxide copolymers, tyrosine-derived polycarbonate, polyglycolide, modified cellulose, collagen, poly(hydroxybutyrate), polyanhydride, polyphosphoester, poly(amino acids), chitosan or combinations and/or derivatives . In the example of Figure 6, each set of flaps 80, 82 includes a pair of flaps, although it is to be understood that in other embodiments there may be a single flap 80 and a single flap 82, while in other embodiments there may be more than two flaps in each set 80, 82. This will be dependent upon the specific design of the closure element, the size of the pouch and so on. These are considerations which a person skilled in the art will readily be able to arrive at from common general knowledge.

With reference to Figure 6A, the distal end of the catheter 12 is shown passing through the valve assembly formed by the flap sets 80, 82 and into the chamber 50 of the pouch 40. The arrangement shown in Figure 6A is consistent with that when the pouch 40 is fully fitted over the distal end 14 of the sheath 12, as shown in Figure 5 in particular. In this regard, it will be appreciated that the outwardly extending flap set 80 will be housed within the lumen of the holding sheath 60 and yet be able to slid relative to the sheath 60 and the catheter 12.

Figure 6B shows the movement of the flaps of the valve assembly as the distal end 14 of the catheter 12 slides out of the pouch 40. This can be seen, the internal flaps 82 will first begin to close, by the natural resilience of the flap/pouch structure. When the distal end 14 of the catheter 12 is fully removed from the pouch 14, as shown in Figures 6C and 6D, the outwardly extending flaps 80 will also close, in the opposite direction from the inner flaps 82, thereby providing a second closure barrier. The combination of the flap sets 80, 82 will ensure that there is a barrier both to material flowing out of the chamber 50 of the pouch 40 and also of material into the chamber 50 of the pouch 40.

The skilled person will appreciate that Figure 6 depicts one example of valve arrangement and that other valve arrangements using one or more valve leaflets could be used. For example, only one of the valve sets 80,82 may be provided in some embodiments.

With reference now to Figures 7 and 8, these show another example of closure mechanism suitable for the apparatus taught herein. Figure 7 shows a cross-sectional view of the outlet 26 of the catheter 12 and, disposed circumferentially around this, the open proximal end 42 of the pouch 40. As can be seen, the wall of the catheter 12 keeps the proximal pouch end 42 open, such that the lumen 15 of the catheter 12 is in fluid communication with the pouch chamber 50.

Referring now to Figure 8, this shows the arrangement of Figure 7 once the catheter 12 has been removed from the pouch. The proximal end 42 of the pouch 40 has an element of elastomeric material, which forms a closure wall 46 closing off the open end 42 of the pouch 40. In practice, the closure wall 46 may leave a small aperture 48, typically as a result of it not being possible for the elastomeric material to contract completely. However, the small aperture 48 can readily be small enough not to allow any significant egress or ingress of material into and out of the pouch 40. The closure wall 46 may be formed of the same material as the pouch 40 or may be formed of any other suitable highly elastomeric material, for instance polyurethane, polyether block amide, polysiloxane rubber, latex or hydrogels.

Figures 9 and 10 show another example of valve which could be used in place of the valve arrangements shown in Figures 6 to 8, at the open end 42 of the pouch 40. Referring first to Figure 9, the valve 84 is a disk shaped valve made of any of the materials discussed above. The valve 84 includes, in this embodiment, three radial slits which extend from the centre point of the valve 84 towards the circumferential periphery of the disk and radially equally spaced, so as to form three equal valve leaflets 88. Figure 9 shows the valve 84 in its closed configuration, in which the leaflets 88 lie in the plane of the valve 84 and seal the valve closed. Figure 10 shows the valve 84 in an open configuration, as it would be when the pouch 40 is fitted over the catheter 12. In the configuration of Figure 10, the leaflet 88 are biased out of the plane of the valve 84, typically to curve upwardly or downwardly, in dependence on the direction of bias applied to the valve leaflets 88. When opened in this manner, the leaflets provide an opening 87 through the middle of the valve.

The skilled person will appreciate that there are many other kinds of suitable valves.

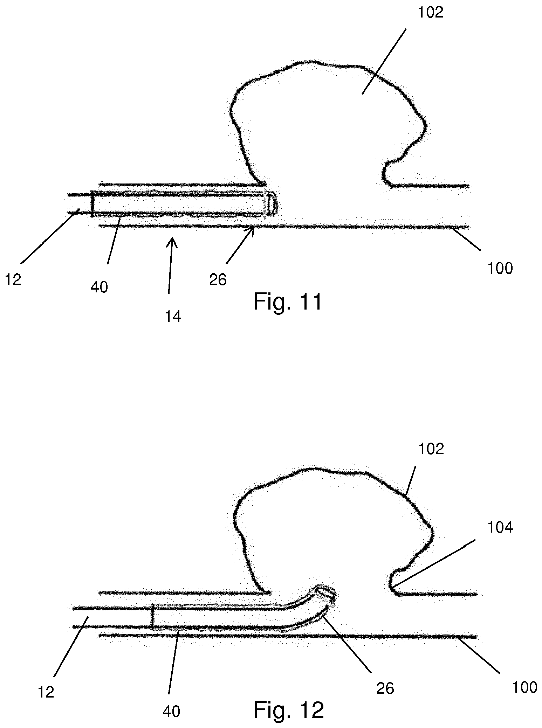

Referring now to Figures 11 to 15, these show the use of the apparatus of Figures 1 to 6 in filling a saccular aneurysm 102 within a vessel 100 of a patient. In this example, the aneurysm sac 102 is formed in a side of the vessel. It is to be appreciated that Figures 11 to 15, for the sake of clarity, do not depict the sheath 60 fitted to the catheter 12 to overlie the proximal portion of the pouch 40. The sheath 60, though, is part of the apparatus shown in Figures 11 to 15 and the sequence shown in these Figures depicts how the assembly will behave with the sheath present.

The catheter assembly 12 is passed endoluminally from a remote percutaneous entry point in the patient, until the distal end 14 of the assembly 10 reaches the location of the aneurysm 102. During the endoluminal passage of the assembly 12, the distal end 14 of the catheter 12 may be used in a non-steered configuration and allowed to curve with the curvature of the vessels. It is not excluded, however, that the steering mechanism 16 could be operated to bend the distal end 14 through curves in the patient's vasculature and into branch vessels.

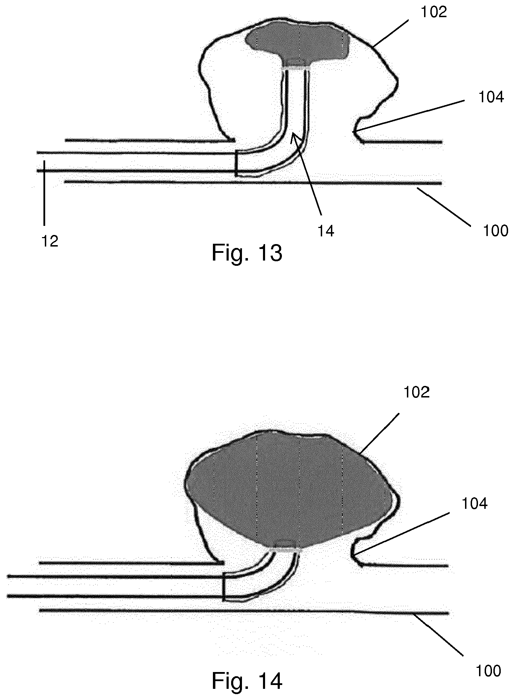

Once the distal end 14 reaches the neck 104 of the aneurysm sac 100, the steering mechanism 16 is operated so as to curve the distal end 14 through the neck 104 into the aneurysm sac 100. The tip 14 can then be pushed further into the aneurysm sac 100, until the tip 26 and its outlet are deemed sufficiently far into the volume of the sac 100. This may be adjacent the vessel wall. With reference to Figure 13, filler material is then fed, from a source 30, through the lumen of the catheter 12 to the outlet port 26. The filler material flows into the chamber 50 of the pouch 40, causing the pouch 40 to expand radially outwardly and into the aneurysm sac 102. The overlying sheath 60 prevents all but the distal portion of the pouch 40 from expanding radially outwards, thereby ensuring that the pouch 40 expands from its distal end and, as a result, that this occurs within the aneurysm sac 102. As filler material continues to be pumped into the pouch 40, the pressure of filler material within the pouch 40 will cause the pouch 40 to begin to slide along the catheter 12 and progressively out of the sheath 60. In addition, this progressive filling of the pouch 40 can follow a sequence of filling the saccular aneurysm from its deepest point (relative to the neck 104) towards the neck 104. This filling sequence can be seen from a comparison of Figures 13 to 15.

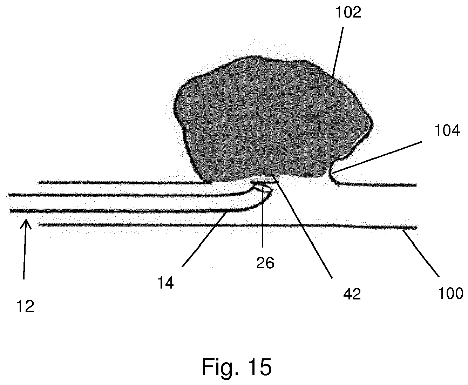

With reference now to Figure 14, this shows the pouch expanded to an extent that the major part of the volume of the saccular aneurysm has been filled. Only a small portion of the aneurysm sac 102, adjacent the neck 104, remains to be filled. As can be seen in Figure 15, once the pouch 40 has been completely filled with filler material, the pouch 40 will become detached form the catheter 12, and in practice the closure mechanism will actively close off the opening of the proximal end 42, in a manner as shown, for instance in Figures 6 to 10. The catheter 12 can at this step be completely removed from within the patient. The conical section between the proximal sleeve end 64 and the main section of the sheath 60 assists in avoiding snagging of the apparatus within the patient's vasculature as the catheter 12 is withdrawn.

The apparatus and its method of use ensure reliable positioning into a saccular aneurysm (or other body portion) and avoids the risk of incorrect pouch positioning, incorrect pouch filling and also seepage of filler material out of the aneurysm 102.

A variety of different fillers can be used. In some examples, the filler may be in the form of a solution which contains the necessary ingredients to form a gel or solid, with the solution having sufficient flowability to be able to be fed through the catheter 12 and then, once in the pouch, to become gradually more viscous. An example of a suitable material is a cyanoacrylate. In other embodiments, the filler could be in two parts, the first part being pre-deposited within the chamber 50 of the pouch 40. This may, for example, be a dry part of the filler, which will then react or swell when a fluid is introduced into the pouch (for instance a saline solution) through the catheter. Suitable examples of such a filler are an alginate or a super absorbent polymer such as a polymethylmethacrylate or derivative thereof. Other suitable examples of filler material include: an acrylic glue suitable for neurological applications, such as GluBran; two-component glues mixed outside the patient and then fed through the catheter 12 from the supply 30 prior to curing; a two-component glue mixed once both components are within the pouch 40; a highly viscous hydrophobic liquid, such as silicone, fat and so on; coagulated blood which may be triggered by alcohol or by a thrombin; a gelatine sponge such as GelFoam, polyethylene glycol diacrylate, or any other suitable material; and combinations of any of the materials disclosed herein.

The two part filler combinations can include synthetic superabsorbant polymers, such as polyacrylamides and polyacrylates, which absorb water; polysaccharides (and derivatives, including semi-synthetics where the synthetic parts are grafted onto the polysaccharide), such as alginate, which absorb water; and modified proteins, which again absorb water. Thus, the solid or semi-solid part may be disposed in the pouch and water supplied through the delivery mechanism to expand the filler in the pouch.

The closure membrane 46 may be made of any suitable bio-compatible elastic material, preferably having low creep.

Although in the preferred embodiment the sheath 60 is flexible and able to curve with the distal end 14 of the catheter 12, in some embodiments it may be rigid or substantially rigid, in which case the catheter is steerable at its proximal tip portion 22 only.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number 1700991.1, from which this application claims priority, and in the abstract accompanying this application are incorporated herein by reference.

## Claims

1. Apparatus for filling a cavity in a patient, the apparatus including:
an elongate catheter having a catheter distal end and a tip at an extremity of the catheter distal end, at least a part of the catheter distal end being steerable;
a steering mechanism connected to the steerable part of the catheter distal end; and
a pouch having an open proximal pouch end, a closed distal pouch end, a pouch length between the proximal and distal pouch ends, and a pouch wall forming a pouch chamber, the pouch along its length being slidably disposed in coaxial overlying relationship on the catheter distal end with the distal pouch end being adjacent and overlying the catheter tip, the pouch being steerable with the catheter distal end, the pouch being separable from the catheter;
the catheter having a lumen extending to the catheter distal end and in communication with the pouch chamber.

2. Apparatus according to claim 1, including a sheath attached to the catheter and extending over at least a part of the pouch on the catheter.

3. Apparatus according to claim 2, wherein the sheath includes a sheath proximal end and a sheath distal end, wherein the sheath proximal end is attached to the catheter and the sheath distal end extends over the pouch.

4. Apparatus according to claim 3, wherein the sheath distal end is longitudinally spaced from the catheter tip.

5. Apparatus according to claim 3 or 4, wherein the sheath distal end terminates at least 1cm from the catheter tip.

6. Apparatus according to any one of claims 2 to 5, wherein the sheath is formed of a flexible material.

7. Apparatus according to any preceding claim, wherein the proximal end of the pouch is sealable in a substantially closed state by a valve including one or valve leaflets or an elastic membrane.

8. Apparatus according to any preceding claim, wherein the pouch is formed of one or more of:
biodegradable material;
a material which promotes cell growth;
an inelastic material.

9. Apparatus according to any preceding claim, including a filler material.

10. Apparatus according to claim 9, wherein the filler material is a biodegradable material and/or promotes cell growth.

11. Apparatus according to claim 9 or 10, wherein the filler material is curable at body temperature.

12. Apparatus according to any one of claims 9 to 11, wherein the filler material is in the form of a solution or other liquid deliverable to the pouch chamber through the lumen of the steerable catheter.

13. Apparatus according to any one of claims 9 to 12, wherein the filler material comprises at least one of: cyanoacrylate, alginate, a super absorbent polymer such as a polymethylmethacrylate or derivative thereof, an acrylic glue a two-component glue, a highly viscous hydrophobic liquid, coagulated blood, a gelatine sponge, polyethylene glycol diacrylate.

14. Apparatus according to any one of claims 9 to 13, wherein the filler material includes a first material located within the pouch chamber.

15. Apparatus according to claim 14, wherein the first material is in dry form.

16. Apparatus according to claim 14 or 15, wherein the first material includes at least one of alginate and a super absorbent polymer.

17. Apparatus according to claim 16, wherein the superabsorbent polymer is polymethylmethacrylate or a derivative thereof.

18. Apparatus for filling a cavity in a patient, the apparatus including:
an elongate catheter having a catheter distal end and a tip at an extremity of the catheter distal end;
a pouch having an open proximal pouch end, a closed distal pouch end and a pouch chamber, the pouch being disposed in coaxial overlying relationship on the catheter distal end with the distal pouch end adjacent the catheter tip;
the catheter having a lumen extending to the catheter distal end and in communication with the pouch chamber; and
a sheath attached to the catheter and extending over at least a part of the pouch on the catheter.
